# EUROPEAN PATENT APPLICATION

(11) **EP 4 079 397 A1**
(43) Date of publication of application: **26.10.2022**
(21) Application number: 20903555.9
(22) Date of filing: 14.09.2020
(51) Int. Cl.: B01J 8/22, B01J 4/02, C12M 1/40, C07K 1/04

(54) **SYNTHESIS DEVICE AND SYNTHESIS METHOD**

(30) Priority: 18.12.2019 JP 2019228421
(71) Applicant: Toray Engineering Co., Ltd., Tokyo 103-0028 (JP)
(72) Inventor: TSUDA, Yuichiro, Otsu-shi, Shiga 520-0842 (JP)
(74) Representative: Global IP Europe Patentanwaltskanzlei
(86) International application number: PCT/JP2020/034738
(87) International publication number: WO 2021/124627

(57) **Abstract**

[OBJECT] The present invention relates to a synthesis device for chemically synthesizing a protein, a peptide, a nucleic acid, or the like in a reaction vessel provided with carriers, and it is an object thereof to reduce the number of carriers that will not readily react in the reaction vessel, and thereby improve the yield of the target product that is obtained by chemical synthesis.

[MEANS OF SOLVE THE PROBLEMS] A synthesis device 1 comprises a reaction vessel 9 that contains a large number of carriers B and to which a solution L is supplied, and a gas supply means 20 for stirring the solution L and the carrier B by supplying gas to the reaction vessel 9.

## Description

### [TECHNICAL FIELD]

The present invention relates to a synthesis device and a synthetic method for chemically synthesizing proteins, peptides, nucleic acids, and the like.

### [BACKGROUND ART]

One method for chemically synthesizing proteins, peptides, nucleic acids, and the like is to sequentially supply a plurality of types of solution (reagent) to a reaction vessel, and allow the reaction to proceed in the reaction vessel. For example, in the case of synthesizing a nucleic acid, a large number of granular carriers (beads) are provided in a reaction vessel, detritylation, coupling, oxidation, and capping are repeatedly performed while sequentially supplying solutions to the reaction vessel, and bases are bonded one after another from the carriers.

Dozens of kinds of solution may be used here, and these solutions are selectively sent to the reaction vessel, and the target substance (nucleic acid) is produced by the molecular material contained in the solutions. One known device for performing such chemical synthesis is, for example, the synthesis device described in

### Patent Literature 1.

### [PRIOR ART DOCUMENT]

### [PATENT LITERATURE]

[Patent Literature 1] Japanese Laid-Open Patent Application Publication No. 2019-170187

### [SUMMARY OF THE INVENTION]

### [PROBLEMS TO BE SOLVED BY THE INVENTION]

A large number of carriers are contained in the reaction vessel provided to the synthesis device. A solution is supplied into the reaction vessel through a port provided at either the upper and lower part of the reaction vessel, and the solution that has passed through the reaction vessel is discharged from another port provided the other end. Chemical synthesis is made to proceed by sequentially changing the solution that is passed through the reaction vessel.

However, some of the carriers may not readily react, depending on their position in the reaction vessel. That is, even though the solution passes through the reaction vessel, the solution may not reach those carriers located near the side wall surfaces of the reaction vessel, for example. In this case, chemical synthesis at the carrier will not occur sufficiently. As a result, the yield of the obtained target product (nucleic acid) may be lower.

In view of this, it is an object of the present invention to reduce the number of carriers that do not readily react in the reaction vessel, and to improve the yield of the target product obtained by chemical synthesis.

### [MEANS TO SOLVE THE PROBLEMS]

The synthesis device disclosed herein comprises a reaction vessel that contains a large number of carriers and to which a solution is supplied, and a gas supply means for stirring the solution and the carriers by supplying a gas to the reaction vessel.

With this synthesis device, there are fewer carriers that do not readily react depending on their location in the reaction vessel, which makes it possible to improve the yield of the target product obtained by chemical synthesis.

The conventional approach has been to fill up the reaction vessel with carriers and pass the solutions through the reaction vessel.

However, with the invention disclosed herein, a gas is supplied into the reaction vessel.

In view of this, the reaction vessel preferably has a vessel upper surface located higher than an upper surface of a layer of the carriers and the solution in a state in which the carriers and a required amount of solution are in place.

With this configuration, the carriers and the required amount of solution are held in the reaction vessel, and a space is formed in the upper part of the reaction vessel. Therefore, when the gas is supplied into the reaction vessel, the solution and the carriers can flow so as to escape into the space at the top part the vessel, so the solution and the carriers are stirred more briskly.

The carriers move vigorously when the gas is supplied into the reaction vessel, and if some of the carriers cling to the upper surface of the reaction vessel, it is possible that those carriers will not make contact with the solution.

Therefore, preferably, the reaction vessel has an internal filter that is located higher than an upper surface of a layer of the carriers in a state prior to the supply of the gas to the reaction vessel, and is located lower than a height of a liquid surface of the solution containing the gas supplied to the reaction vessel, the internal filter being permeable to the solution and impermeable to the carriers.

When an internal filter is provided at such a position, even if the carriers move and cling to the internal filter, they will be mixed with the solution. This prevents some of the carriers from clinging to the upper surface of the reaction vessel and being unable to make contact with the solution.

Also, preferably, the gas supply means is configured to transport the solution outside the reaction vessel by pushing by the gas through a pipe connected to the reaction vessel, and supply the gas used to transport the solution directly to the reaction vessel to stir the carriers and the solution by the gas when the transport of the solution to the reaction vessel by the gas is complete.

In this case, the gas transporting the solution to the reaction vessel is used to stir the carriers and the solution. If a mechanism is provided for transporting the solution to the reaction vessel by means of a gas, it may not be necessary for a gas supply mechanism for stirring to be separately provided, and this simplifies the configuration of the synthesis device.

Also, preferably, the reaction vessel has at its upper part an inlet through which passes the gas used to discharge the solution to outside of the reaction vessel, and has at its lower part an outlet through which the solution is discharged.

With this configuration, if a gas is supplied from the upper part of the reaction vessel, the used solution will be discharged from the lower part of the reaction vessel.

Also, the invention disclosed herein is a synthesis method in which chemical synthesis is performed by supplying a solution to a reaction vessel containing a large number of carriers, the method including a supply step of supplying a required amount of the solution to the reaction vessel containing the carriers, and a synthesis step of chemically synthesizing, while stirring the solution and the carriers, by supplying a gas to the reaction vessel.

With this synthesis method, there are fewer carriers that do not readily react depending on their location in the reaction vessel, which makes it possible to improve the yield of the target product obtained by chemical synthesis.

### [EFFECTS OF THE INVENTION]

According to the invention disclosed herein, there are fewer carriers that do not readily react depending on their location in the reaction vessel, which makes it possible to improve the yield of the target product obtained by chemical synthesis.

### [BRIEF DESCRIPTION OF THE DRAWINGS]

FIG. 1 is a configuration diagram showing an example of a synthesis device;
FIG. 2 is a schematic view of a reaction vessel;
FIG. 3 is an explanatory diagram showing a state in which a solution has been supplied to a reaction vessel provided with carriers;
FIG. 4 is an explanatory diagram showing a state in which gas is being supplied to the reaction vessel;
FIG. 5A is an explanatory diagram showing a modification example of the reaction vessel;
FIG. 5B is an explanatory diagram showing a modification example of the reaction vessel; and
FIG. 6 is a flowchart illustrating a synthesis method.

### [EMBODIMENTS TO CARRY OUT THE INVENTION]

### Overall Configuration of Synthesis Device

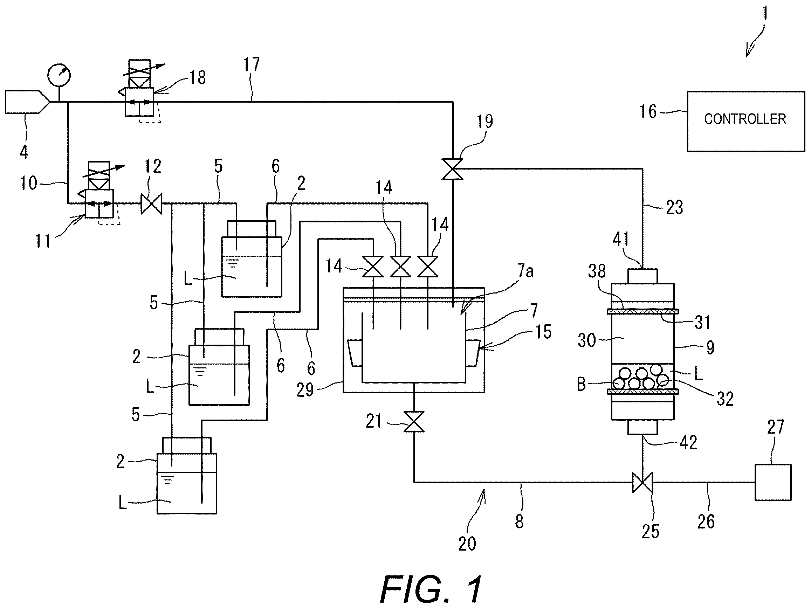

FIG. 1 is a configuration diagram showing an example of a synthesis device. The synthesis device 1 shown in FIG. 1 is a device for chemically synthesizing proteins, peptides, nucleic acids, and the like. The synthesis device 1 comprises a reaction vessel 9. A plurality of types of solution (reagent) L are sequentially supplied to the reaction vessel 9, and chemical synthesis is allowed to proceed in the reaction vessel 9. When synthesizing a nucleic acid, a large number of granular carriers B are provided inside the reaction vessel 9. The carriers B are also called beads, and are made of glass or a polymer, for example. While the solution L is sequentially supplied to the reaction vessel 9, detritylation, coupling, oxidation, and capping are repeatedly performed, and bases (molecular materials) are bonded one after another from the carriers B. There are dozens of types of solution L that can be used, and these solutions L are selectively sent to the reaction vessel 9, and the target product (nucleic acid) is produced by the molecular materials contained in the solutions L.

The synthesis device 1 comprises an area for providing storage containers (reagent bottles) 2 in a number equal to the number of solutions L used. The solutions L are stored in the respective storage containers 2. In FIG. 1, only three storage containers 2 are shown, and the other storage containers 2 are not depicted. Each storage container 2 is a sealed container, and an introduction pipe 5 and an outlet pipe 6 are connected to each one.

The synthesis device 1 comprises a tank 4 for storing gas, the introduction pipes 5, the outlet pipes 6, an intermediate container 7, an intermediate pipe 8, the reaction vessel 9, a measuring mechanism 15, and a control device 16. The tank 4 is filled with a gas whose pressure is higher than atmospheric pressure, and in the synthesis device 1 disclosed herein, the tank is filled with argon gas (an inert gas). Although argon gas is used in this embodiment, any other gas may be used as long as it is an inert gas and is dehydrated. As described below, this gas is used to transport the solutions L of the storage containers 2 to the reaction vessel 9, as well as to stir the carriers B and the solution L in the reaction vessel 9.

The introduction pipes 5, whose number is equal to that of the plurality of storage containers 2, branch off from a shared upstream pipe 10. A regulator 11 and a valve 12 are provided to the upstream pipe 10. The upstream pipe 10 is connected to the tank 4, the gas in the tank 4 is supplied to the storage containers 2, and the internal pressure of the storage containers 2 is adjusted by the regulator 11. The internal pressure of the storage containers 2 is increased by the gas, which causes the solution L in the storage containers 2 to be pumped out through the outlet pipes 6. That is, the solution L in each storage container 2 is fed under pressure to the intermediate container 7 through the outlet pipe 6 due to the differential pressure between the storage container 2 and the intermediate container 7.

A valve 14 is provided to each of the outlet pipes 6. By selecting which of the valves 14 is in an open state, a specific solution L from among the solutions L in the plurality of storage containers 2 is selectively sent through an outlet pipe 6 to the intermediate container 7. The selection of the valve 14 to be in an open state is performed by the control device 16.

The intermediate container 7 is used for metering each solution L. The intermediate container 7 is a bottomed cylindrical container in which each solution L can be held. The plurality of outlet pipes 6 are gathered together at the inlet region (opening 7a) of the intermediate container 7. Therefore, the solution L selectively sent through an outlet pipe 6 is introduced into the intermediate container 7 and held in this intermediate container 7.

The measuring mechanism 15 measures the solution L stored in the intermediate container 7. In the measuring mechanism 15, the intermediate container 7 functions as a measuring container. The measuring mechanism 15 has, for example, a measuring device featuring a load cell or the like, and measures the solution L held in the intermediate container 7. The weight obtained by the weighing mechanism 15 is transmitted to the control device 16. The control device 16 controls the opening and closing operation of the valve 14 on the basis of the measurement result to acquire the specified amount of solution L in the intermediate container 7. The specified amount of solution L is sent through the intermediate pipe 8 to the reaction vessel 9. A valve 21 is provided to the intermediate pipe 8, and the valve 21 is in a closed state when weighing is being performed.

The method for supplying the solution L from the intermediate container 7 to the reaction vessel 9 is pressure feeding with a gas, and the gas in the tank 4 is used for this. During this pressure feeding, the valve 21 is in an open state. For this pressure feeding, the synthesis device 1 comprises a sealed container 29 in which the intermediate container 7 is housed. A gas pipe 17 is provided between the sealed container 29 and the tank 4. A second regulator 18 and a valve 19 are provided to the gas pipe 17. The valve 19 shown in FIG. 1 is a three-way valve, but may be some other type.

The intermediate container 7 is open in the sealed container 29, and when the gas in the tank 4 is supplied to the sealed container 29, the pressure (internal pressure) of the gas in the sealed container 29 acts on the solution L held in the intermediate container 7, and the solution L in the intermediate container 7 is pressure-fed through the intermediate pipe 8 to the reaction vessel 9 by the differential pressure between the sealed container 29 (intermediate container 7) and the reaction vessel 9. Thus, with the synthesis device 1 disclosed herein, the method of feeding the solution L from the intermediate container 7 to the reaction vessel 9 is pumping under gas pressure, and this pumping is performed by a gas supply means 20. With the configuration shown in FIG. 1, the gas supply means 20 includes the tank 4, the gas pipe 17, the sealed container 29, and the intermediate pipe 8. However, the configuration of the gas supply means 20 may be different from this.

Because of this, a solution L is selectively sent to the intermediate container 7 from one or more of the storage containers 2, and when the measurement is performed in the intermediate container 7, that solution L is sent to the reaction vessel 9. The solution L is used for chemical synthesis in the reaction vessel 9. After this, the solution L is discharged through the discharge pipe 26 to a waste liquid tank 27.

This supply of the solution L to the reaction vessel 9 is repeated for each type of solution L so that a plurality of types of solution L are sequentially supplied to the reaction vessel 9, and chemical synthesis is conducted in the reaction vessel 9. A large number of carriers B are provided in the reaction vessel 9, and bases are bonded one after another from the carriers B to synthesize a nucleic acid.

### Reaction Vessel 9

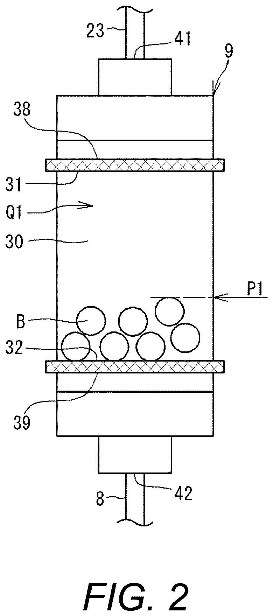

FIG. 2 is a schematic view of the reaction vessel 9. The reaction vessel 9 shown in FIG. 2 is constituted by a vessel that is longer in the vertical direction than in the horizontal direction. A large number of carriers B are provided inside the reaction vessel 9. In FIG. 2, etc., the carriers B are shown larger than the actual carriers B to facilitate explanation, and the number thereof shown is also smaller than in reality. The reaction vessel 9 may have a form other than what is shown in FIG. 2, and may be a vessel that is longer in the horizontal direction than in the vertical direction.

The intermediate pipe 8 is connected to the lower part of the reaction vessel 9. A pipe 23 branching off from the gas pipe 17 (see FIG. 1) is connected to the upper part of the reaction vessel 9. Inside the reaction vessel 9, an upper filter 38 is provided at the upper part, and a lower filter 39 is provided at the lower part. The upper filter 38 is a net-like member that is permeable to the solution L but impermeable to the carriers B, and is a member that covers the internal space of the reaction vessel 9 from above. The lower filter 39 is a net-like member that is permeable to the solution L but impermeable to the carriers B, and is a member that covers the internal space of the reaction vessel 9 from below. With the reaction vessel 9 disclosed herein, the lower surface of the upper filter 38 is defined as the vessel upper surface 31, and the upper surface of the lower filter 39 is defined as the vessel lower surface 32. The space between the vessel upper surface 31 and the vessel lower surface 32 is a reaction space 30, and a large number of the carriers B are provided in this reaction space 30.

As shown in FIG. 2, the carriers B do not completely fill the reaction space 30, and instead are provided up to a first height position P1 located part of the way up in the vertical direction of the reaction space 30. Therefore, this produces a space Q1 in the reaction vessel 9 above the layer of the carriers B.

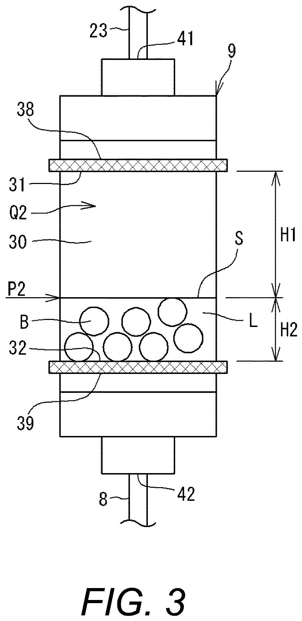

FIG. 3 is an explanatory diagram showing a state in which the solution L has been supplied to the reaction vessel 9 containing the carriers B. The solution L is supplied through the intermediate pipe 8, from the lower side of the reaction vessel 9. Various kinds of solution L are supplied to the reaction vessel 9, as mentioned above. Regardless of which solution L is supplied, the solution L does not fill the reaction vessel 9. That is, the solution L is not supplied until the reaction space 30 is full, but rather is supplied up to a second height position P2 located part of the way up in the vertical direction of the reaction space 30. This height position P2 is based on the supplied solution L and the carriers B that have been provided in advance. Therefore, inside the reaction vessel 9, this produces a space Q2 above the layer (mixed layer) of the carriers B and the solution L.

Of the reaction space 30, the volume of the space Q2 may be larger than the combined volume of the carriers B and the solution L. With the reaction vessel 9 disclosed herein, the cross-sectional area of the reaction space 30 is constant in the vertical direction, so the height H1 of the space Q2 is greater than the height H2 of the layer between the carriers B and the solution L.

Here, the method for supplying the solution L to the reaction vessel 9 will be described again. As discussed above, the method for sending the solution L to the reaction vessel 9 is pressure feeding by means of the gas in the tank 4 (see FIG. 1), and this pressure feeding is performed by the gas supply means 20. The solution L sent to the reaction vessel 9 is a solution that has been measured by the measuring mechanism 15. That is, the amount of the solution L supplied to the reaction vessel 9 is controlled, and as discussed above (see FIG. 3), this results in a state in which the solution L has been supplied up to the second height position P2 part of the way up in the reaction space 30 in the vertical direction.

Therefore, the volume of the reaction vessel 9 (reaction space 30) is set large. More specifically, the volume of the reaction vessel 9 (reaction space 30) is set large enough that even if the preset maximum amount of the solution L is supplied to the reaction vessel 9, the height of the liquid surface of the solution L will be at the second height position P2 part of the way up in the vertical direction of the reaction space 30. That is, in a state where the required amount of solution L has been supplied to the reaction vessel 9 containing the carriers B, the volume of the reaction vessel 9 will be greater than the combined volume of the required amount of solution L and the carriers B. To put this yet another way, in a state in which the carriers B and the required amount of the solution L are held, the vessel upper surface 31 of the reaction vessel 9 is located higher than the upper surface S of the layer of the carriers B and the solution L. With the synthesis device 1 disclosed herein, as mentioned above, the vessel upper surface 31 is the lower surface of the upper filter 38.

The volume of the reaction vessel 9 is greater than the volume of the supplied solution L. For example, from the standpoint of stirring efficiency, the volume of the reaction vessel 9 is preferably at least 2.5 times the volume (maximum required amount) of the solution L, and is also preferably no more than 4 times the volume of the solution L so as not to make the reaction vessel 9 any larger than necessary.

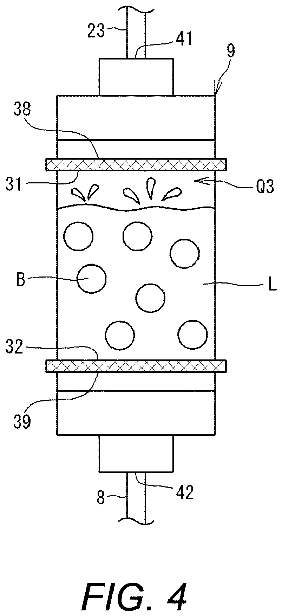

FIG. 4 is an explanatory diagram showing a state in which gas is being supplied to the reaction vessel 9. When the solution L is supplied to the reaction vessel 9 containing the carriers B, the solution L and the carriers B are agitated by this supply of gas to the reaction vessel 9. In particular, with the synthesis device 1 disclosed herein (see FIG. 1), the solution L measured by the measuring mechanism 15 is pushed through the intermediate pipe 8 connected to the reaction vessel 9 by the gas of the tank 4, and conveyed to the reaction vessel 9 . This conveyance is performed by the gas supply means 20, as mentioned above. When the conveyance of the solution L to the reaction vessel 9 by the gas is complete, the gas used to convey the solution L is supplied directly to the reaction vessel 9. Therefore, as shown in FIG. 4, the carriers B and the solution L can be agitated by the gas inside the reaction vessel 9. That is, the gas for stirring the carriers B and the solution L is supplied by the gas supply means 20.

The supply of gas for stirring is continued for a specific length of time. As shown in FIGS. 3 to 4, once the required amount of solution L has been supplied to the reaction vessel 9, the state of stirring with the gas is continued. In the case of the synthesis device 1 disclosed herein, the solution L is not supplied to the reaction vessel 9 while the gas is being supplied to the reaction vessel 9 for stirring. Some of the gas supplied to the reaction vessel 9 is discharged to the outside of the reaction vessel 9 (outside of the synthesis device 1) through the pipe 23.

As described above (see FIG. 3), inside the reaction vessel 9, the space Q2 is produced above the layer (mixed layer) of the carriers B and the solution L. Therefore, even when gas is added to the reaction vessel 9 for stirring, the carriers B and the solution L including the gas will not reach (are unlikely to reach) the vessel upper surface 31. That is, as shown in FIG. 4, even when gas is supplied into the reaction vessel 9, a space Q3 is produced at the upper part of the container in the reaction space 30, and the solution L and the carriers B can flow so as to escape to the space Q3 at the upper part of the vessel. Therefore, the gas briskly stirs the carriers B and the solution L.

The supply of gas produces a mixed flow of the solution L and the carriers B, containing the gas, inside the reaction vessel 9. The vessel upper surface 31 is located higher than the height of the upper surface of this mixed flow.

A plurality of carriers B may be present as a clump in the reaction vessel 9. In this case, the carriers B at the center of this clump will be less likely to come into contact with the solution L. However, in the invention disclosed herein, clumping is eliminated by the stirring, that is, the carriers B are dispersed in the solution L, and more of the carriers B come into contact with the solution L.

A modification example of the reaction vessel 9 will now be described.

As discussed above (see FIG. 4), even if gas is supplied to the reaction vessel 9 for stirring, the carriers B and the solution L including the gas will not reach (are unlikely to reach) the vessel upper surface 31. However, when the gas is supplied to the reaction vessel 9, the carriers B may move so vigorously that some of the carriers B may stick to the vessel upper surface 31. In this event, those carriers B may not be able to come into contact with the solution L and may not contribute to the production of the target substance (nucleic acid).

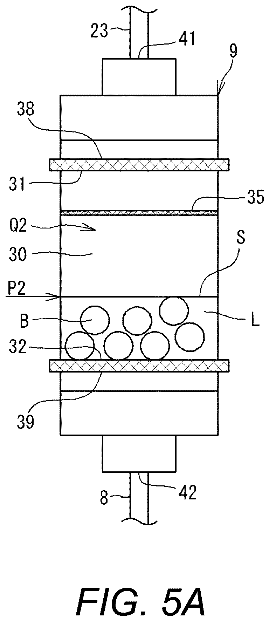

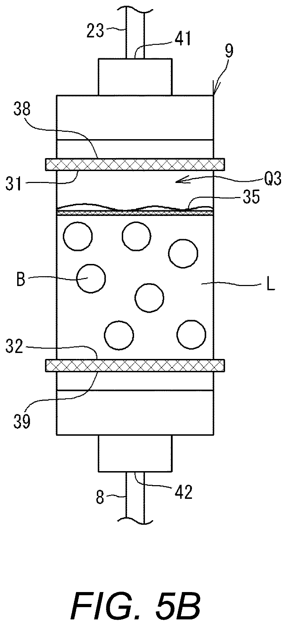

In view of this, as shown in FIGS. 5A and 5B, the reaction vessel 9 may have an internal filter 35. FIGS. 5A and 5B are explanatory views showing a modification example of the reaction vessel 9. Similar to the upper filter 38 and the lower filter 39, the internal filter 35 is constituted by a mesh-like member that is permeable to the solution L but is impermeable to the carriers B. As shown in FIG. 5A, this internal filter 35 is provided at a position higher than the upper surface of the layer of the carriers B in a state before the gas has been supplied to the reaction vessel 9. Furthermore, as shown in FIG. 5B, the internal filter 35 is provided at a position lower than the height of the liquid surface of the solution L containing the gas in a state in which the gas has been supplied to the reaction vessel 9.

Since the internal filter 35 is provided at the position described above, the carriers B will not stick to the vessel upper surface 31. Furthermore, even if the carriers B should move and stick to the internal filter 35, they can be mixed with the solution L, and those carriers B will also contribute to the production of the target substance (nucleic acid). Thus, when the reaction vessel 9 has the internal filter 35, it is possible to prevent some of the carriers B from sticking to the vessel upper surface 31 of the reaction vessel 9 and being unable to come into contact with the solution L. When the internal filter 35 is provided, the upper filter 38 may be omitted.

Whether the reaction vessel 9 is as shown in FIGS. 2, 3 and 4 (first mode), or the reaction vessel 9 is as shown in FIGS. 5A and 5B (second mode), once the chemical reaction with the solution L has taken place for a specific length of time, the solution L is discharged to the outside of the reaction vessel 9. The means for this discharge is as follows.

First, the stirring is halted in the reaction vessel 9. To this end, the supply of gas to the reaction vessel 9 is stopped. The gas valve 19 provided along the gas pipe 17 (see FIG. 1) is switched so that the gas in the tank 4 flows through the pipe 23 connected to the reaction vessel 9. Along with the switching of the valve 19, the valve 25 provided along the intermediate pipe 8 is also switched so that the reaction vessel 9 is connected to the discharge pipe 26. Consequently, the gas in the tank 4 is supplied to the reaction vessel 9, and the used solution L in the reaction vessel 9 is pushed by the gas and discharged from the discharge pipe 26 into the waste liquid tank 27.

In order to discharge the solution L in the reaction vessel 9, the gas in the tank 4 is supplied to the reaction vessel 9 from the upper part of the vessel. Therefore, even if some of the carriers B stick to the vessel upper surface 31 (upper filter 38) or some of the carriers B stick to the internal filter 35 (see FIG. 5A), those carriers B can be knocked down to the bottom of the reaction vessel 9.

As shown in FIG. 1, the reaction vessel 9 has an upper port 41 and a lower port 42. The pipe 23 branching off from the gas pipe 17 is connected to the upper port 41, and the intermediate pipe 8 is connected to the lower port 42. When the solution L is discharged from the reaction vessel 9, the upper port 41 serves as a gas inlet and the lower port 42 serves as a solution L discharge port. That is, the reaction vessel 9 has at its upper part an inflow port (upper port 41) through which passes the gas for discharging the solution L to the outside of the reaction vessel 9. The reaction vessel 9 has at its lower part a discharge port (lower port 42) through which the solution L is discharged as a waste liquid. When the solution L is supplied to the reaction vessel 9, and when the gas for stirring is supplied, the valves 19 and 25 are switched so that the lower port 42 becomes the inflow port of the solution L and the gas.

### Synthesis Device 1 Disclosed Herein

As described above, the synthesis device 1 in each of the modes described above comprises a reaction vessel 9 and a gas supply means 20 capable of supplying gas to this reaction vessel 9. The reaction vessel 9 contains a large number of carriers B and is supplied with the solution L used for chemical synthesis. The gas supply means 20 stirs the solution L and the carriers B by supplying gas to the reaction vessel 9 during chemical synthesis in the reaction vessel 9. With this synthesis device 1, the carriers B are dispersed in the solution L in the reaction vessel 9, allowing the solution L to come into contact with all of the many carriers B. As a result, fewer of the carriers B are unlikely to react than in the past. For example, carriers B that have stuck to the side wall surface of the reaction vessel 9 can move to the center of the reaction vessel 9, or carriers B that have clumped together can be dispersed, allowing more of the carriers B to come into contact with the solution L. As a result, it is possible to improve the yield of the target substance (nucleic acid) obtained by chemical synthesis.

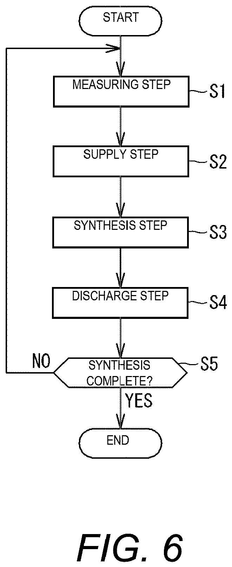

The synthesis method performed by the synthesis device 1 in each of the above-mentioned mode, that is, the synthesis method for performing chemical synthesis by supplying a solution L to the reaction vessel 9 containing a large number of carriers B, includes the following steps. FIG. 6 is a flowchart illustrating this synthesis method.
Supply step S2: A step of supplying a required amount of solution L to the reaction vessel 9 containing the carriers B (see FIG. 3).
Synthesis step S3: A step of performing chemical synthesis while stirring the solution L and the carriers B by supplying gas to the reaction vessel 9 (see FIG. 4).

In the case of the synthesis device 1 disclosed herein, in the supply step S2, the solution L is supplied to the reaction vessel 9 by pressure feeding a gas.

In the synthesis step S3, when the gas is supplied to the reaction vessel 9, the gas supply parameters, such as the pressure and the flow rate of the gas, are controlled by the control device 16. This control is preferably performed by adjusting the supply parameters according to the type of solution L. The gas supply parameters when the solution L is supplied to the reaction vessel 9 in the supply step S2, and the gas supply parameters when the gas is supplied to the reaction vessel 9 for stirring in the synthesis step S3, may be the same or may be different.

Furthermore, the synthesis method disclosed herein (see FIG. 6) also includes the following steps.
Measuring step S1: A step of measuring the solution L to be supplied to the reaction vessel 9.
Discharge step S4: A step of discharging the solution L used for chemical synthesis in the reaction vessel 9.
Determination step S5: A step of determining whether or not all the synthesis is complete.

In the measuring step S1, the required amount of solution L is measured. In the discharge step S4, as described above, if gas is supplied from the upper part (upper port 41) of the reaction vessel 9, the used solution L is discharged as waste liquid from the lower part (lower port 42) of the reaction vessel 9.

In the determination step S5, if it is determined that the synthesis is not complete, the processing proceeds to the measurement step S1, and the next measurement of the solution L is performed. After this, the supply step S2, the synthesis step S3, and the discharge step S4 are repeated until all the synthesis is complete.

The synthesis method disclosed herein is not a method in which the solution L is circulated by using the reaction vessel 9 as one of the flow paths, and is instead a method in which a mixed flow of the solution L and the carriers B is produced inside the reaction vessel 9 by the gas supplied to the reaction vessel 9. It is therefore possible to reduce the amount of solution L used, and the chemical synthesis also takes less time.

With the synthesis method disclosed herein, gas is supplied for stirring to the reaction vessel 9 containing the carriers B and the solution L. Therefore, a flow of the solution L, the carriers B, and the gas is produced in the reaction vessel 9, and the volume of the contents of the reaction vessel 9 is greater than that before the supply of gas.

In view of this, in the supply step S2 (see FIG. 3), the carriers B and the required amount of the solution L are held in the reaction vessel 9, and in this state, the vessel upper surface 31 of the reaction vessel 9 is set to be located higher than the upper surface of the layer of the carriers B and the solution L.

Consequently, the carriers B and the required amount of solution L are held in the lower part of the reaction vessel 9, and the space Q2 is formed in the upper part of the reaction vessel 9. Therefore, in the subsequent synthesis step S3, when gas is supplied to the reaction vessel 9 (see FIG. 4), the solution L and the carriers B can flow so as to escape to the space at the upper part of the vessel, and the solution L and the carriers B can be stirred more vigorously.

If the reaction vessel 9 were filled with the carriers B and the solution L, then when gas was supplied to the reaction vessel 9 from below for the purpose of stirring, the solution L would end up flowing out of the pipe 23 connected to the upper part of the reaction vessel 9. However, as described above, the solution L can be prevented from flowing out of the pipe 23 by setting the volume of the reaction vessel 9 such that the upper surface 31 of the reaction vessel 9 is higher than the upper surface of the layer of the carriers B and the solution L.

Also, in the case of the synthesis method disclosed herein, in the supply step S2, the solution L measured by the measuring mechanism 15, that is, a solution L that is outside of the reaction vessel 9, is pushed by gas through the intermediate pipe 8 connected to the reaction vessel 9, and conveyed to the reaction vessel 9. Then, when the conveyance of the solution L to the reaction vessel 9 by the gas is complete, in the synthesis step S3, the gas that was used to convey the solution L is supplied directly to the reaction vessel 9, and the carriers B and the solution L are stirred by this gas. The conveyance of the solution L by gas to the reaction vessel 9 and its stirring by the gas are performed by the gas supply means 20. As discussed above, the gas that conveys the solution L to the reaction vessel 9 is used to stir the carriers B and the solution L. If a mechanism for conveying the solution L to the reaction vessel 9 by gas is provided, there will be no need to separately provide a gas supply mechanism for stirring, and the configuration of the synthesis device 1 can be simplified.

### Other

With the synthesis device 1, the gas used to pressure-feed the solution L from the storage container 2 to the measuring mechanism 15, the gas used to pressure-feed the solution L from the measuring mechanism 15 to the reaction vessel 9, and the gas used to stir the carriers B and the solution L in the reaction vessel 9 are all supplied from the same tank 4. However, although not depicted in the drawings, the configuration may instead be such that these gases are supplied from different tanks (gas sources). For example, a gas used for pressure-feeding and a gas used for stirring may be supplied from different tanks (gas sources). Also, with the above-mentioned synthesis device 1, the flow path for supplying the solution L to the reaction vessel 9 and the flow path for supplying the gas for stirring are the same (both use the intermediate pipe 8), but the flow path for supplying the solution L to the reaction vessel 9 and the flow path for supplying the gas for stirring to the reaction vessel 9 may be different. Also, the reaction vessel 9 may have a shape other than what is shown in the drawings.

In the above disclosure, in the reaction vessel 9, a gas is supplied from the lower part of the reaction vessel 9 for stirring. However, although not depicted in the drawings, an internal pipe may be provided so as to extend from the upper part of the reaction vessel 9 into the layer of the carriers B and the solution L, and gas may be supplied through this internal pipe into the layer for stirring. Here again, chemical synthesis can be performed while stirring the solution L and the carriers B.

The embodiments disclosed herein are merely examples in all respects, and are not intended to be restrictive. The technical scope of the present invention is not limited to the above embodiments, and the technical scope encompasses all modifications within a scope equivalent to the configuration described in the claims.

### [DESCRIPTION OF THE REFERENCE NUMERALS]

1: synthesis device
8: intermediate pipe (pipe connecting to the reaction vessel)
9: reaction vessel
20: gas supply means
31: vessel upper surface
35: internal filter
41: upper port (inflow port)
42: lower port (discharge port)
B: carriers
L: solution

## Claims

1. A synthesis device, comprising:
a reaction vessel that contains a large number of carriers and to which a solution is supplied; and
a gas supply means for stirring the solution and the carriers by supplying a gas to the reaction vessel.

2. The synthesis device according to claim 1, wherein
the reaction vessel has a vessel upper surface located higher than an upper surface of a layer of the carriers and the solution in a state in which the carriers and a required amount of the solution are in place.

3. The synthesis device according to claim 1 or 2, wherein
the reaction vessel has an internal filter that is located higher than an upper surface of a layer of the carriers in a state prior to the supply of the gas to the reaction vessel, and is located lower than a height of a liquid surface of the solution containing the gas supplied to the reaction vessel, the internal filter being permeable to the solution and impermeable to the carriers.

4. The synthesis device according to any one of claims 1 to 3, wherein
the gas supply means is configured to
transport the solution outside the reaction vessel by pushing by the gas through a pipe connected to the reaction vessel, and
supply the gas used to transport the solution directly to the reaction vessel to stir the carriers and the solution by the gas when the transport of the solution to the reaction vessel by the gas is complete.

5. The synthesis device according to any one of claims 1 to 4, wherein
the reaction vessel has at its upper part an inlet through which passes the gas used to discharge the solution to outside of the reaction vessel, and has at its lower part an outlet through which the solution is discharged.

6. A synthesis method in which chemical synthesis is performed by supplying a solution to a reaction vessel containing a large number of carriers, the method including:
a supply step of supplying a required amount of the solution to the reaction vessel containing the carriers; and
a synthesis step of chemically synthesizing while stirring the solution and the carriers by supplying a gas to the reaction vessel.
